# EUROPEAN PATENT APPLICATION

(11) **EP 1 978 103 A1**
(43) Date of publication of application: **08.10.2008**
(21) Application number: 07006891.1
(22) Date of filing: 03.04.2007
(51) Int. Cl.: C12Q 1/68

(54) **Method and kits for detection of EGFRvIII**

(71) Applicant: Bergen Teknologioverforing AS, 5006 Bergen (NO)
(72) Inventor: Prestegarden, Lars, 5053 Bergen (NO)
(74) Representative: Kröncke, Rolf

(57) **Abstract**

The present invention relates to methods for the detection of EGFRvIII nucleic acid molecules. In particular, the present invention relates to PCR based methods for the detection of EGFRvIII in biological samples. In a further aspect, the present invention relates to detection kits for detecting EGFRvIII in biological samples. The present invention allows to specifically identify expression of EGFRvIII nucleic acid molecules, in particular, of EGFRvIII RNA in individuals. Said methods and kits are particularly useful to determine and identify precancerous or cancerous lesions at early stages in individuals suspected to have cancer.

SEQUENCE LISTING

<110>  Bergen Teknologioverforing AS

<120>  Method and kits for the detection of EGFRvIII

<130>  4200-1EP-1

<160>  9

<170>  PatentIn version 3.1

<210>  1
<211>  5525
<212>  DNA
<213>  Homo sapiens

<400>  1
gccggagtcc cgagctagcc ccggcggccg ccgccgccca gaccggacga caggccacct      60
cgtcggcgtc cgcccgagtc cccgcctcgc cgccaacgcc acaaccaccg cgcacggccc     120
cctgactccg tccagtattg atcgggagag ccggagcgag ctcttcgggg agcagcgatg     180
cgaccctccg ggacggccgg ggcagcgctc ctggcgctgc tggctgcgct ctgcccggcg     240
agtcgggctc tggaggaaaa gaaagtttgc caaggcacga gtaacaagct cacgcagttg     300
ggcacttttg aagatcattt tctcagcctc cagaggatgt tcaataactg tgaggtggtc     360
cttgggaatt tggaaattac ctatgtgcag aggaattatg atctttcctt cttaaagacc     420
atccaggagg tggctggtta tgtcctcatt gccctcaaca cagtggagcg aattcctttg     480
gaaaacctgc agatcatcag aggaaatatg tactacgaaa attcctatgc cttagcagtc     540
ttatctaact atgatgcaaa taaaaccgga ctgaaggagc tgcccatgag aaatttacag     600
gaaatcctgc atggcgccgt gcggttcagc aacaaccctg ccctgtgcaa cgtggagagc     660
atccagtggc gggacatagt cagcagtgac tttctcagca acatgtcgat ggacttccag     720
aaccacctgg gcagctgcca aaagtgtgat ccaagctgtc ccaatgggag ctgctggggt     780
gcaggagagg agaactgcca gaaactgacc aaaatcatct gtgcccagca gtgctccggg     840
cgctgccgtg gcaagtcccc cagtgactgc tgccacaacc agtgtgctgc aggctgcaca     900
ggcccccggg agagcgactg cctggtctgc cgcaaattcc gagacgaagc cacgtgcaag     960
gacacctgcc ccccactcat gctctacaac cccaccacgt accagatgga tgtgaacccc    1020

gagggcaaat acagctttgg tgccacctgc gtgaagaagt gtccccgtaa ttatgtggtg 1080
acagatcacg gctcgtgcgt ccgagcctgt ggggccgaca gctatgagat ggaggaagac 1140
ggcgtccgca agtgtaagaa gtgcgaaggg ccttgccgca aagtgtgtaa cggaataggt 1200
attggtgaat ttaaagactc actctccata aatgctacga atattaaaca cttcaaaaac 1260
tgcacctcca tcagtggcga tctccacatc ctgccggtgg catttagggg tgactccttc 1320
acacatactc ctcctctgga tccacaggaa ctggatattc tgaaaaccgt aaaggaaatc 1380
acagggtttt tgctgattca ggcttggcct gaaaacagga cggacctcca tgcctttgag 1440
aacctagaaa tcatacgcgg caggaccaag caacatggtc agttttctct tgcagtcgtc 1500
agcctgaaca taacatcctt gggattacgc tccctcaagg agataagtga tggagatgtg 1560
ataatttcag gaaacaaaaa tttgtgctat gcaaatacaa taaactggaa aaaactgttt 1620
gggacctccg gtcagaaaac caaaattata agcaacagag gtgaaaacag ctgcaaggcc 1680
acaggccagg tctgccatgc cttgtgctcc cccgagggct gctggggccc ggagcccagg 1740
gactgcgtct cttgccggaa tgtcagccga ggcagggaat gcgtggacaa gtgcaacctt 1800
ctggagggtg agccaaggga gtttgtggag aactctgagt gcatacagtg ccacccagag 1860
tgcctgcctc aggccatgaa catcacctgc acaggacggg gaccagacaa ctgtatccag 1920
tgtgcccact acattgacgg cccccactgc gtcaagacct gcccggcagg agtcatggga 1980
gaaaacaaca ccctggtctg gaagtacgca gacgccggcc atgtgtgcca cctgtgccat 2040
ccaaactgca cctacggatg cactgggcca ggtcttgaag gctgtccaac gaatgggcct 2100
aagatcccgt ccatcgccac tgggatggtg ggggccctcc tcttgctgct ggtggtggcc 2160
ctggggatcg gcctcttcat gcgaaggcgc cacatcgttc ggaagcgcac gctgcggagg 2220
ctgctgcagg agagggagct tgtggagcct cttacaccca gtggagaagc tcccaaccaa 2280
gctctcttga ggatcttgaa ggaaactgaa ttcaaaaaga tcaaagtgct gggctccggt 2340
gcgttcggca cggtgtataa gggactctgg atcccagaag gtgagaaagt taaaattccc 2400
gtcgctatca aggaattaag agaagcaaca tctccgaaag ccaacaagga aatcctcgat 2460
gaagcctacg tgatggccag cgtggacaac ccccacgtgt gccgcctgct gggcatctgc 2520
ctcacctcca ccgtgcagct catcacgcag ctcatgccct tcggctgcct cctggactat 2580
gtccgggaac acaaagacaa tattggctcc cagtacctgc tcaactggtg tgtgcagatc 2640
gcaaagggca tgaactactt ggaggaccgt cgcttggtgc accgcgacct ggcagccagg 2700
aacgtactgg tgaaaacacc gcagcatgtc aagatcacag attttgggct ggccaaactg 2760
ctgggtgcgg aagagaaaga ataccatgca gaaggaggca aagtgcctat caagtggatg 2820
gcattggaat caattttaca cagaatctat acccaccaga gtgatgtctg gagctacggg 2880
gtgactgttt gggagttgat gacctttgga tccaagccat atgacggaat ccctgccagc 2940
gagatctcct ccatcctgga gaaaggagaa cgcctccctc agccacccat atgtaccatc 3000
gatgtctaca tgatcatggt caagtgctgg atgatagacg cagatagtcg cccaaagttc 3060

cgtgagttga tcatcgaatt ctccaaaatg gcccgagacc cccagcgcta ccttgtcatt 3120
caggggggatg aaagaatgca tttgccaagt cctacagact ccaacttcta ccgtgccctg 3180
atggatgaag aagacatgga cgacgtggtg gatgccgacg agtacctcat cccacagcag 3240
ggcttcttca gcagcccctc cacgtcacgg actcccctcc tgagctctct gagtgcaacc 3300
agcaacaatt ccaccgtggc ttgcattgat agaaatgggc tgcaaagctg tcccatcaag 3360
gaagacagct tcttgcagcg atacagctca gaccccacag gcgccttgac tgaggacagc 3420
atagacgaca ccttcctccc agtgcctgaa tacataaacc agtccgttcc caaaaggccc 3480
gctggctctg tgcagaatcc tgtctatcac aatcagcctc tgaaccccgc gcccagcaga 3540
gacccacact accaggaccc ccacagcact gcagtgggca accccgagta tctcaacact 3600
gtccagccca cctgtgtcaa cagcacattc gacagccctg cccactgggc ccagaaaggc 3660
agccaccaaa ttagcctgga caaccctgac taccagcagg acttctttcc caaggaagcc 3720
aagccaaatg gcatctttaa gggctccaca gctgaaaatg cagaatacct aagggtcgcg 3780
ccacaaagca gtgaatttat tggagcatga ccacggagga tagtatgagc cctaaaaatc 3840
cagactcttt cgatacccag gaccaagcca cagcaggtcc tccatcccaa cagccatgcc 3900
cgcattagct cttagaccca cagactggtt ttgcaacgtt tacaccgact agccaggaag 3960
tacttccacc tcgggcacat tttgggaagt tgcattcctt tgtcttcaaa ctgtgaagca 4020
tttacagaaa cgcatccagc aagaatattg tccctttgag cagaaattta tctttcaaag 4080
aggtatattt gaaaaaaaaa aaaagtatat gtgaggattt ttattgattg gggatcttgg 4140
agtttttcat tgtcgctatt gatttttact tcaatgggct cttccaacaa ggaagaagct 4200
tgctggtagc acttgctacc ctgagttcat ccaggcccaa ctgtgagcaa ggagcacaag 4260
ccacaagtct tccagaggat gcttgattcc agtggttctg cttcaaggct tccactgcaa 4320
aacactaaag atccaagaag gccttcatgg ccccagcagg ccggatcggt actgtatcaa 4380
gtcatggcag gtacagtagg ataagccact ctgtcccttc ctgggcaaag aagaaacgga 4440
ggggatggaa ttcttcctta gacttacttt tgtaaaaatg tccccacggt acttactccc 4500
cactgatgga ccagtggttt ccagtcatga gcgttagact gacttgtttg tcttccattc 4560
cattgttttg aaactcagta tgctgcccct gtcttgctgt catgaaatca gcaagagagg 4620
atgacacatc aaataataac tcggattcca gcccacattg gattcatcag catttggacc 4680
aatagcccac agctgagaat gtggaatacc taaggatagc accgcttttg ttctcgcaaa 4740
aacgtatctc ctaatttgag gctcagatga aatgcatcag gtcctttggg gcatagatca 4800
gaagactaca aaaatgaagc tgctctgaaa tctcctttag ccatcacccc aaccccccaa 4860
aattagtttg tgttacttat ggaagatagt tttctccttt tacttcactt caaaagcttt 4920
ttactcaaag agtatatgtt ccctccaggt cagctgcccc caaaccccct ccttacgctt 4980
tgtcacacaa aaagtgtctc tgccttgagt catctattca agcacttaca gctctggcca 5040
caacagggca ttttacaggt gcgaatgaca gtagcattat gagtagtgtg gaattcaggt 5100

agtaaatatg aaactagggt ttgaaattga taatgctttc acaacatttg cagatgtttt 5160
agaaggaaaa aagttccttc ctaaaataat ttctctacaa ttggaagatt ggaagattca 5220
gctagttagg agcccacctt ttttcctaat ctgtgtgtgc cctgtaacct gactggttaa 5280
cagcagtcct ttgtaaacag tgttttaaac tctcctagtc aatatccacc ccatccaatt 5340
tatcaaggaa gaaatggttc agaaaatatt ttcagcctac agttatgttc agtcacacac 5400
acatacaaaa tgttcctttt gcttttaaag taatttttga ctcccagatc agtcagagcc 5460
cctacagcat tgttaagaaa gtatttgatt tttgtctcaa tgaaaataaa actatattca 5520
tttcc 5525

<210> 2
<211> 4724
<212> DNA
<213> homo sapiens

<400> 2
gccggagtcc cgagctagcc ccggcggccg ccgccgccca gaccggacga caggccacct 60
cgtcggcgtc cgcccgagtc cccgcctcgc cgccaacgcc acaaccaccg cgcacggccc 120
cctgactccg tccagtattg atcgggagag ccggagcgag ctcttcgggg agcagcgatg 180
cgaccctccg ggacggccgg ggcagcgctc ctggcgctgc tggctgcgct ctgcccggcg 240
agtcgggctc tggaggaaaa gaaaggtaat tatgtggtga cagatcacgg ctcgtgcgtc 300
cgagcctgtg gggccgacag ctatgagatg gaggaagacg gcgtccgcaa gtgtaagaag 360
tgcgaagggc cttgccgcaa agtgtgtaac ggaataggta ttggtgaatt taaagactca 420
ctctccataa atgctacgaa tattaaacac ttcaaaaact gcacctccat cagtggcgat 480
ctccacatcc tgccggtggc atttaggggt gactccttca cacatactcc tcctctggat 540
ccacaggaac tggatattct gaaaaccgta aaggaaatca cagggttttt gctgattcag 600
gcttggcctg aaaacaggac ggacctccat gcctttgaga acctagaaat catacgcggc 660
aggaccaagc aacatggtca gttttctctt gcagtcgtca gcctgaacat aacatccttg 720
ggattacgct ccctcaagga gataagtgat ggagatgtga taatttcagg aaacaaaaat 780
ttgtgctatg caaatacaat aaactggaaa aaactgtttg ggacctccgg tcagaaaacc 840
aaaattataa gcaacagagg tgaaaacagc tgcaaggcca caggccaggt ctgccatgcc 900
ttgtgctccc ccgagggctg ctggggcccg gagcccaggg actgcgtctc ttgccggaat 960
gtcagccgag gcagggaatg cgtggacaag tgcaaccttc tggagggtga gccaagggag 1020
tttgtggaga actctgagtg catacagtgc cacccagagt gcctgcctca ggccatgaac 1080
atcacctgca caggacgggg accagacaac tgtatccagt gtgcccacta cattgacggc 1140
ccccactgcg tcaagacctg cccggcagga gtcatgggag aaaacaacac cctggtctgg 1200

aagtacgcag acgccggcca tgtgtgccac ctgtgccatc caaactgcac ctacggatgc 1260
actgggccag gtcttgaagg ctgtccaacg aatgggccta agatcccgtc catcgccact 1320
gggatggtgg gggccctcct cttgctgctg gtggtggccc tggggatcgg cctcttcatg 1380
cgaaggcgcc acatcgttcg gaagcgcacg ctgcggaggc tgctgcagga gagggagctt 1440
gtggagcctc ttacacccag tggagaagct cccaaccaag ctctcttgag gatcttgaag 1500
gaaactgaat tcaaaaagat caaagtgctg ggctccggtg cgttcggcac ggtgtataag 1560
ggactctgga tcccagaagg tgagaaagtt aaaattcccg tcgctatcaa ggaattaaga 1620
gaagcaacat ctccgaaagc caacaaggaa atcctcgatg aagcctacgt gatggccagc 1680
gtggacaacc cccacgtgtg ccgcctgctg ggcatctgcc tcacctccac cgtgcagctc 1740
atcacgcagc tcatgccctt cggctgcctc ctggactatg tccgggaaca caaagacaat 1800
attggctccc agtacctgct caactggtgt gtgcagatcg caaagggcat gaactacttg 1860
gaggaccgtc gcttggtgca ccgcgacctg gcagccagga acgtactggt gaaaacaccg 1920
cagcatgtca agatcacaga ttttgggctg gccaaactgc tgggtgcgga agagaaagaa 1980
taccatgcag aaggaggcaa agtgcctatc aagtggatgg cattggaatc aattttacac 2040
agaatctata cccaccagag tgatgtctgg agctacgggg tgactgtttg ggagttgatg 2100
acctttggat ccaagccata tgacggaatc cctgccagcg agatctcctc catcctggag 2160
aaaggagaac gcctccctca gccacccata tgtaccatcg atgtctacat gatcatggtc 2220
aagtgctgga tgatagacgc agatagtcgc ccaaagttcc gtgagttgat catcgaattc 2280
tccaaaatgg cccgagaccc ccagcgctac cttgtcattc agggggatga aagaatgcat 2340
ttgccaagtc ctacagactc caacttctac cgtgccctga tggatgaaga agacatggac 2400
gacgtggtgg atgccgacga gtacctcatc ccacagcagg gcttcttcag cagcccctcc 2460
acgtcacgga ctcccctcct gagctctctg agtgcaacca gcaacaattc caccgtggct 2520
tgcattgata gaaatgggct gcaaagctgt cccatcaagg aagacagctt cttgcagcga 2580
tacagctcag accccacagg cgccttgact gaggacagca tagacgacac cttcctccca 2640
gtgcctgaat acataaacca gtccgttccc aaaaggcccg ctggctctgt gcagaatcct 2700
gtctatcaca atcagcctct gaaccccgcg cccagcagag acccacacta ccaggacccc 2760
cacagcactg cagtgggcaa ccccgagtat ctcaacactg tccagcccac ctgtgtcaac 2820
agcacattcg acagccctgc ccactgggcc cagaaaggca gccaccaaat tagcctggac 2880
aaccctgact accagcagga cttctttccc aaggaagcca agccaaatgg catctttaag 2940
ggctccacag ctgaaaatgc agaataccta agggtcgcgc cacaaagcag tgaatttatt 3000
ggagcatgac cacggaggat agtatgagcc ctaaaaatcc agactctttc gatacccagg 3060
accaagccac agcaggtcct ccatcccaac agccatgccc gcattagctc ttagacccac 3120
agactggttt tgcaacgttt acaccgacta gccaggaagt acttccacct cgggcacatt 3180
ttgggaagtt gcattccttt gtcttcaaac tgtgaagcat ttacagaaac gcatccagca 3240

agaatattgt ccctttgagc agaaatttat ctttcaaaga ggtatatttg aaaaaaaaaa 3300
aaagtatatg tgaggatttt tattgattgg ggatcttgga gtttttcatt gtcgctattg 3360
atttttactt caatgggctc ttccaacaag gaagaagctt gctggtagca cttgctaccc 3420
tgagttcatc caggcccaac tgtgagcaag gagcacaagc cacaagtctt ccagaggatg 3480
cttgattcca gtggttctgc ttcaaggctt ccactgcaaa acactaaaga tccaagaagg 3540
ccttcatggc cccagcaggc cggatcggta ctgtatcaag tcatggcagg tacagtagga 3600
taagccactc tgtcccttcc tgggcaaaga agaaacggag gggatggaat tcttccttag 3660
acttactttt gtaaaaatgt ccccacggta cttactcccc actgatggac cagtggtttc 3720
cagtcatgag cgttagactg acttgtttgt cttccattcc attgttttga aactcagtat 3780
gctgcccctg tcttgctgtc atgaaatcag caagagagga tgacacatca aataataact 3840
cggattccag cccacattgg attcatcagc atttggacca atagcccaca gctgagaatg 3900
tggaatacct aaggatagca ccgcttttgt tctcgcaaaa acgtatctcc taatttgagg 3960
ctcagatgaa atgcatcagg tcctttgggg catagatcag aagactacaa aaatgaagct 4020
gctctgaaat ctcctttagc catcacccca accccccaaa attagtttgt gttacttatg 4080
gaagatagtt ttctcctttt acttcacttc aaaagctttt tactcaaaga gtatatgttc 4140
cctccaggtc agctgccccc aaaccccctc cttacgcttt gtcacacaaa aagtgtctct 4200
gccttgagtc atctattcaa gcacttacag ctctggccac aacagggcat tttacaggtg 4260
cgaatgacag tagcattatg agtagtgtgg aattcaggta gtaaatatga aactagggtt 4320
tgaaattgat aatgctttca caacatttgc agatgtttta gaaggaaaaa agttccttcc 4380
taaaataatt tctctacaat tggaagattg gaagattcag ctagttagga gcccaccttt 4440
tttcctaatc tgtgtgtgcc ctgtaacctg actggttaac agcagtcctt tgtaaacagt 4500
gttttaaact ctcctagtca atatccaccc catccaattt atcaaggaag aaatggttca 4560
gaaaatattt tcagcctaca gttatgttca gtcacacaca catacaaaat gttccttttg 4620
cttttaaagt aatttttgac tcccagatca gtcagagccc ctacagcatt gttaagaaag 4680
tatttgattt ttgtctcaat gaaaataaaa ctatattcat ttcc 4724

<210> 3
<211> 8
<212> DNA
<213> homo sapiens

<220>
<221> misc_feature
<222> (1)..(1)

<223>  n represents a sequence of 0 to 46 nucleic acids which correspond
      s to the sequence of EGFR exon 1 upstream of the sequence gaaag a
      t the 3' end of exon 1 of EGFR

<220>

<221>  misc_feature

<222>  (8)..(8)

<223>  n represents a sequence of 0 to 46 nucleic acids which correspond
      s to the sequence of EGFR exon 8 downstream of the first nucleic
      acid g at the 5' end of exon 8 of EGFR

<400>  3
ngaaaggn                                                                 8

<210>  4

<211>  8

<212>  DNA

<213>  homo sapiens

<220>

<221>  misc_feature

<222>  (8)..(8)

<223>  n represents a sequence of 0 to 46 nucleic acids which correspond
      s to the reverse sequence of EGFR exon 1 downstram of the sequenc
      e ct at the 3' end of reverse exon 1 of EGFR

<220>

<221>  misc_feature

<222>  (1)..(1)

<223>  n represents a sequence of 0 to 46 nucleic acids which correspond
      s to the reverse sequence of EGFR exon 8 upstream of the sequence
       ttac at the 3' end of reverse exon 8 of EGFR

<400>  4
nttacctn                                                                 8

<210>  5

<211>  20

<212>  DNA

<213>  homo sapiens

<400>  5
gctctggagg aaaagaaagg                                                    20

<210>  6
<211>  20
<212>  DNA
<213>  homo sapiens

<400>  6
gcccttcgca cttcttacac                                                    20

<210>  7
<211>  20
<212>  DNA
<213>  homo sapiens

<400>  7
agctccttca gtccggtttt                                                    20

<210>  8
<211>  20
<212>  DNA
<213>  homo sapiens

<400>  8
gtggttctgg aagtccatcg                                                    20

<210>  9
<211>  20
<212>  DNA
<213>  homo sapiens

<400>  9
gggctctgga ggaaaagaaa                                                    20

## Description

The present invention relates to methods for the detection of EGrRvlll nucleic acid molecules- In particular, the present invention relates to PCR based methods for the detection of EGFRvIII in biological samples. In a further aspect, the present invention relates to detection kits for detecting EGFRvIII in biological samples. The present invention allows to specifically identify expression of EGFRvIII nucleic acid molecules, in particular, of EGFRvIII RNA in individuals. Said methods and kits are particularly useful to determine and identify precancerous or cancerous lesions at early stages in individuals suspected to have cancer.

### Background of the invention

The success of any cancer therapy is based upon its ability to distinguish neoplastic cells from normal cells. Most current chemotherapy or radiotherapy regimens are based upon differential growth rates of tumor cells. In practice, such therapies have been very successful in treating some cancers, but for many other cancers current treatments are either palliative in nature or in the long term are ineffectual. Progress in brain tumor therapy has been especially poor as the survival curve has not appreciably changed in over 60 years. Some progress has been made using biologically based modalities such as harvesting a patient's immune system or therapeutics based upon recent research in molecular biology. However, the specifity of these therapeutics for cancerous cells is poor. Much of the research in biology based therapies has focused on defining tumor specific alterations.

Various molecules are suspected to be connected with the development of tumors and cancer and the detection of mutant and wild type growth factors, oncogenes, and tumor markers has played a critical role for detection and response to therapy in many types of cancer. For example, in oncology the detection of CEA (carcinoembryonic antigen) and PSA (prostate specific antigen) have played a major role in cancer diagnostics.

A related growth factor receptor, the epidermal growth factor receptor (EGFR) is an 170 kD membrane-spanning receptor that regulates differentiation and growth in both normal and neoplastic cells. Elevated levels of EGFR have been reported in many human tumors and cell lines, including breast cancer, adenocarcinoma and squamous lung cancer, gastrointestinal cancers (gastric, colon, pancreatic), renal cell cancer, bladder cancer, glioma, gynaecological carcinomas, and prostate cancer.

Size variant EGFR genes and amplification have been reported in several human cancers. (Hymphry et al., (1988). Amplification and Expression of the Epidermal Growth Factor Receptor Gene in Human Glioma Xenografts. Cancer Research 48:2231-2238; Bigner et al., (1988) J. Neuropathol. Exp. Neurol. 47:191:205; Wong et al., (1987). Increased Expression of the Epidermal Growth Factor Receptor Gene in Malignant Gliomas is Invariably Associated with Gene Amplification. Proc. Natl. Acad. Sci. USA 84:6899-6903. There has been no determination, however, of the molecular basis for the altered EGFR molecules in cells. In 1989, work of Drs. Signer and Vogelstein elucidated the sequence of a EGF receptor mutant that has become known as the type III mutant (also referred to as delta-EGFr or EGFrVIII). This work is described in U.S. Patent Nos. 6,455,498, 6,127,126, 5,981,725, 5,814,317, 5,710,010, 5,401,828 and 5,212,290.

EGFR variants are caused by gene rearrangement accompanied by EGFR gene amplification. There are eight major variants of EGFR that are known: (i) EGFRvI lacks a majority of the extracellular domain of EGFR, (ii) EGFRvII consists of an 83 aa in-frame deletion in the extracellular domain of EGFR, (iii) EGFRvIII consists of a 267 aa in-frame deletion in the extracellular domain of EGFR, (iv) EGFRvIV contains deletions in the cytoplastmic domain of EGFR, (v) EGFRvV contains deletions in cytoplastmic domain of EGFR, (vi) EGFR.TDM/2-7 contains a duplication of exons 2-7 in the extracellular domain of EGFR, (vii) EGFR.TDM/18-25 contains a duplication of exons 18-26 in the tyrosine kinase domain of EGFR, and (viii) EGFR.TDM/18-26 contains a duplication of exons 18-26 in the tyrosine kinase domain of EGFR (Kuan et al. EGF mutant receptor vIII as a molecular target in cancer therapy. Endocr RElat Cancer, 8(2):83-96 (2001). In addition, there is a second, more rare, EGFRvIII mutant (EGFRvIII/Δ12-13) that possesses a second deletion that introduces a novel histidine residue at the junction of exons 11 and 14 (Kuan et al., EGF mutant receptor vIII as a molecular target in cancer therapy. Endocr. Relat Cancer. 8(2):83-96 (2001)).

As mentioned before, the type III mutant EGFR receptor (EGFRvIII) results from an in-frame deletion from joining nucleotides 274 to 1076 in the EGFR cDNA sequence creating a new fusion junction. This in-frame deletion corresponds to a deletion of amino acids 6 to 273 in the extracellular region, which causes constitutive activation of the tyrosine kinase domain. This variant or mutation occurs frequently in ovarian, breast, lung and glioblastoma cancers but has not been reported in normal tissues. Using a polyclonal anti-EGFRvIII-specific antibody detection of this mutant protein in 16 % of non-small cell lung tumors, 78 % of breast carcinomas, 57 % of primary human glial tumors, 86 % of medulloblastoma tumors, and 75 % of ovarian tumors has been reported. Furthermore, the EGFRvIII variant is tumor specific, since detection in any normal tissue examined has not been reported yet. Because EGFRvIII is tumor specific, an assay which can detect and quantify EGFRvIII in urine, serum/plasma, CSF, amniotic fluid, breast, secretions, lung sputum, and tumor cell extracts may be of critical importance in the early detection of various cancers, and also in prognosis, monitoring, and response to therapy. In addition, this assay could serve in the selection of cancer patients for novel mutant EGF-directed anticancer therapies, such as a vaccine, antibody-toxin conjugate, or EGFRvIII-specific tyrosine kinase inhibitors.

Although various methods have been described using the EGFRvIII protein as a target for detection, the detection of nucleic acid molecules corresponding to the mutant EGF receptor EGFRvIII has not been described in the literature. For example WO 2005/010151 describes antibodies directed to the deletion mutants of epidermal growth factor receptor and uses thereof. These antibodies which should specifically identify the EGFRvIII variant but not the wild type EGFR represents a useful diagnostic and therapeutic tool for diagnosing or treating cancer.

US 2005/0222059 discloses EGFRvIII specific monoclonal antibodies as well as ribozymes and their use to detect, treat or prevent EGFRvIII associated cancer, like breast cancer. Recently, Silva H.A.C. et al., European Journal of Cancer, 2006, 42, pages 2617-2622, reported on the molecular detection of EGFRvIII positive cells in the periphal blood of breast cancer patients. This document describes a PCR method for analyzing the expression of the EGFRvIII molecule in a blood sample of breast cancer patients and healthy individuals for determining breast cancer in occult systemic diseases. A nested PCR method is described therein based on using two pairs of primers both allowing amplification of the natural EGFR and the EGFRvIII variant. However, the authors admit that the sensitivity achieved with the method described in the paper was insufficient to allow quantitative PCR.

The problem underlying the present invention is to provide a detection method allowing identification and determination of EGFRvIII nucleic acid molecules in biological samples overcoming the problem known in the art, namely sufficient sensitivity and specificity for the variant form. In particular, specificity is crucial since, typically, the amount of EGFRvIII nucleic acid molecules is extremely low in comparison to the presence of wild type EGFR nucleic acid molecules.

Hence, the present invention is directed to a method for detecting type III mutant EGF receptor (EGFRvIII) in biological samples, a method for detecting cancers at various stages in particular metastasizing cancer and other diseases in biological samples, and a method of assessing treatment and selecting therapy for cancer patients.

### Summary of the present invention

The problems known in the art are solved by providing a method for the detection of EGFRvIII specific nucleic acid molecules in a biological sample. In one embodiment of the present invention, said method is characterized in that one primer of the primer pair used for the amplification of the nucleic acid molecules is an oligonucleotide primer specific for the EGFRvIII variant whereby said primer is spanning exon 1/exon 8 of the EGFR gene, thus, allowing specific amplification of the EGFRvIII variant form only.

In a further aspect, the present invention relates to a nested PCR whereby in the first amplification step the extension time is selected in a way to favour amplification of short nucleic acid fragments.

In addition, another embodiment of the present invention relates to the detection of EGFRvIII specific nucleic acid molecules where the biological sample containing ribonucleic acid molecules is pretreated with RNAse H and a deoxynucleic acid probe specifically hybridizing with a region of exon 2 to exon 7 of the EGFR RNA, namely, the exons deleted in the EGFRvIII variant

In another aspect, the present invention relates to methods for diagnosing or stratifying cancer or precancerous stages in an individual comprising detection of EGFRvIII nucleic acid expression according to the method of the present invention.

The present invention relates to kits for detecting and/or diagnosing EGFRvIII specific nucleic acid molecules in a biological sample.

### Brief description of the drawings

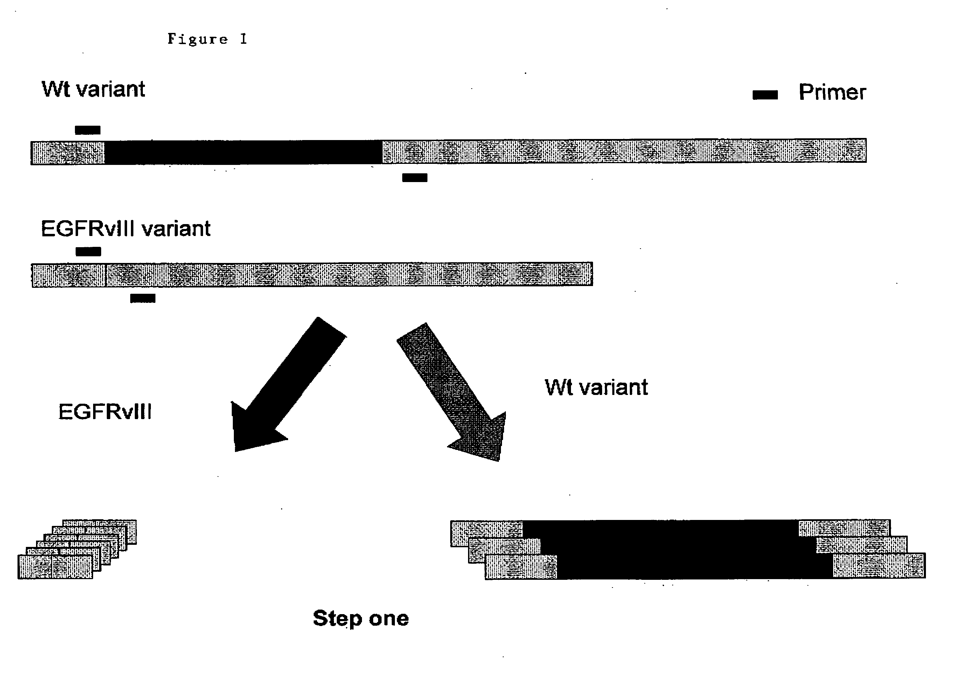

Figure 1 shows step one of a nested PCR based detection method according to the present invention. In the first step wild type EGFR and EGFRvIII variant are amplified. In case of shortening the extension time in the first amplification step to seconds, it is possible to enrich the variant form over the wild type form. Preferably, the primers are located close to the breakpoint which increases the chances of detection since there is always a risk of RNA degradation due to RNAses.

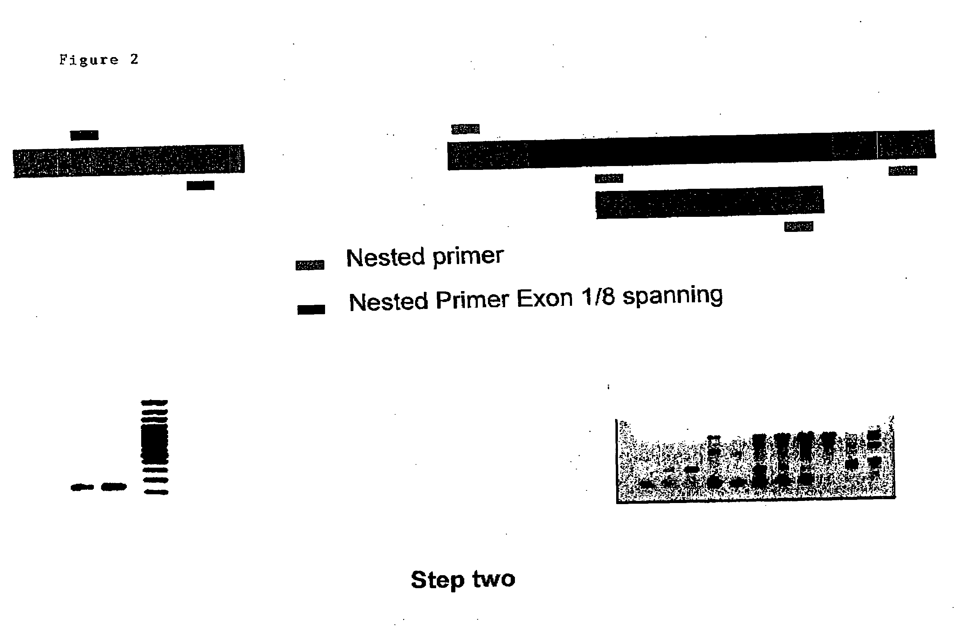

Figure 2 shows step two of the detecting method based on nested PCR technique. On the left side an exon 1/exon 8 spanning forward primer is used allowing variant specific amplification. On the right side of figure 2 the use of a nested primer pair located in exon 1 and exon 8, respectively, is shown resulting in amplification of both forms, the wildtype and the variant form. As shown on the left side only one unambiguous bond is obtained following the approach shown on the right side multiple bands can be detected.
Figure 3 demonstrate the detection of the EGFRvIII variant form in individuals having cancer applying the method according to the present invention.
Figure 4 shows the results obtained from healthy individuals which should not express EGFRvIII variant. As demonstrated in figure 4 no EGFRvIII signal can be detected.

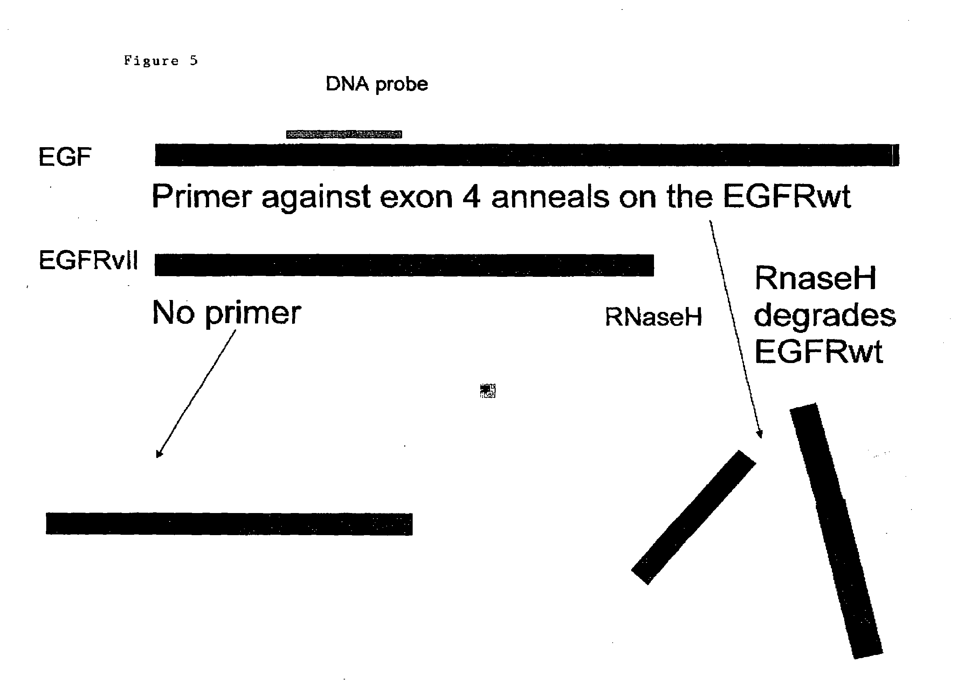

Figure 5 shows the principle of the method of the present invention involving RNAseH treatment. As shown in figure 5 the probe hybridizes to exon 2 to exon 7, here exon 4, of the EGFR wild-type while no hybridization takes place with the RNA of EGFRvIII. RNAse H degrades the DNA/RNA duplexes, in the present case, the wild-type EGFR only while EGFRvIII remains unaffected.
Figure 6 shows the results obtained performing real time PCR with samples obtained from individuals suffering from cancer. As demonstrated all experiments give one clear band which makes automatic detection possible.

### Detailed description of the present invention

The present invention relates to new methods for the detection and, eventually, the diagnosis of cancer. The method according to the present invention is based on the detection of EGFRvIII encoding nucleic acid molecules and allow to differentiate between EGFRvIII nucleic acid molecules and wild type molecules. In one aspect, the specificity for the EGFRvIII variant is achieved by using an oligonucleotide primer spanning exon 1/exon 8 of the EGFR gene in an amplification step, like PCR.

As used herein, the term "individual", "patient", or "subject" which is used herein interchangeably, refers to an individual suspected to be afflicted with cancer. The term "patient", "subject" or "individual" as used herein includes but is not limited to an organism, a mammal including a human, non-human primate, a broadens, horse, cattle or other non-human mammals.

As used herein, the term "lesions" refers to an area of abnormal tissue. A lesion may be benign (noncancerous) or malignant (cancerous).

As used herein, the term "cancer" or "cancerous lesions" refers to diseases in which abnormal cells divide without control. Caner cells can invade nearby tissues and can spread through the bloodstream and lymphatic system to other parts of the body. There are several main types of cancer. Carcinoma is cancer that begins in the skin or in tissues that line or cover internal organs. Sarcoma is cancer that begins in bone, cartilage, gat, muscle, blood vessels, or other connective or supportive tissue. Leukemia is cancer that starts in blood-forming tissue such as the bone marrow, and causes large numbers of abnormal blood cells to be produced and enter the bloodstream. Lymphoma and multiple myeloma are cancers that begin in the cells of the immune system.

Early cancerous lesions or precancerous lesions are lesions that might develop into cancerous lesions, e.g. hyperplastic or dysplastic cells.

The term nucleic acid" as used in the present invention refers to DNA (deoxyribonucleic acid) or RNA (ribonucleic acid) and their single or double stranded polymers (poly nucleotides). Unless limited, the term includes those nucleic acids having similar binding characteristics with the reference nucleic acid and those natural nucleic acid analogues already known and metabolize in a similar way as natural nucleic acids.

The term "same" or "unique" in percentage about two or more nucleotide sequences refers to two or more sequences or sub-sequences which have the same or the same percentage of the nucleotides, while using one of the sequence-comparison algorithms (e.g. Smith-Waterman algorithm) or processing the maximum-corresponding comparison and ranking by visual measure known in the art.

Concerning the sequence-comparison, a section of sequence is taken as a reference for the testing sequence to compare. When applying sequence-comparison algorithm, the testing sequence is entered into the computer as well as the reference one, the sub-sequence coordinate can be assigned if necessary, and the parameters of the sequence algorithm program should be set. The sequence-comparison algorithm can then, based on the program parameters set, calculate the percentage of uniqueness of the testing sequence compared to the reference sequence.

The term "amplification of nucleic acid molecules" or "amplification of DNA or RNA sequence" used in the present invention refers to all methods that allow the amplification of the number of the sample of the targeted nucleotide sequence. The most widely used nucleotide sequence expansion technology is PCR. Basically, it means attaching two primers, which are forward or sense primer and reverse or antisense primer, respectively, to the denaturalized DNA, e.g cDNA, and extending by using thermo-stable DNA polymerase. The later can catalyze RT-PCR (reverse-transcript PCR) and real-time PCR in rapid and repeating cycles. (In a three-step PCR, it means denaturalizationlannealing/extension; in a two-step PCR, it means denaturalization/annealing and extension). RT-PCR can be performed with a single thermo-stable enzyme with reverse-transcription enzymes and DNA polymerase. Optionally, it can also be performed with a single test-tube reaction (Kathy et al., biological technology 17:1034-1036, 1994) containing both enzymes (reverse-transcription enzyme and thermo-stable DNA polymerase).

Polymerase Chain Reaction (PCR) is a repetitive, enzymatic, primed synthesis of a nucleic acid sequence. This procedure is well known and commonly used by those skilled in this art (see Mullis, U.S. Patent Nos. 4,683,195, 4,683,202, and 4,800,159; Saiki et al. (1985) Science 230:1350-1354 and commercial suppliers of reagents for PCR, real time PCR and reverse transcriptase PCR). In the context of the present application, RT-PCR is reverse transcriptase PCR. PCR is based on the enzymatic amplification of a DNA fragment of interest that is flanked by two oligonucleotide primers that hybridize to opposite strands of the target sequence. The primers are oriented with the 3' ends pointing toward each other. Repeated cycles of heat denaturation of the template, annealing of the primers to their complementary sequences, and extension of the annealed primers with a DNA polymerase result in the amplification of the segment defined by the 5' ends of the PCR primers. Since the extension product of each primer can serve as a template for the other primer, each cycle essentially doubles the amount of DNA template produced in the previous cycle. This results in the exponential accumulation of the specific target fragment, up to several million-fold in a few hours. By using a thermostable DNA polymerase such as the Taq polymerase, which is isolated from the thermophilic bacterium *Thermus aquaticus,* the amplification process can be completely automated. Other enzymes which can be used are known to those skilled in the art.

The term "nest polymerase chain reaction (nest PCR or n-PCR) refers to the improved design which can increase the sensitive of PCR, i.e. the secondary PCR which concerns the design of a pair of primers on the PCR product to perform the secondary PCR, the sensitivity can thus increase by 100 to 1000 times.

The term "primer" refers to a short nucleic acid structure, e.g. an oligonucleotide, which can be the starting point of the replication of nucleic acid. While carrying out the PCR reaction, a pair of primers is need, one positive-strand and one negative-strand situated respectively at the both end. The region defined by this pair of primers is the size of the PCR product. The oligonculeotide primer used in the PCR reaction for expanding the desired nucleic acid sequence can be synthesized (e.g. chemically synthesized), for example, by the habitually known chemical method of phosphate-triester or phosphite amide. Primers can also be produced in vitro by the nucleotide sequence expansion method. If the expanded oligonucleotide is double-strand, then it can be converted to a single-strand molecule to protect the single-strand oligonucleotide from the active action of the exonuclease. Furthermore, the primers mentioned in this invention are derived from the recombination plasmid of the insert which has the corresponding nucleotide sequence; the later can be split and retrieved from the selected plasmid by using the proper nucleotide if needed, splitting method such as classification of molecular weight can be used.

In the context of this invention, the term "oligonucleotide" refers to an oligomer or polymer of ribonucleic acid or deoxyribonucleic acid. This term includes oligonucleotides composed of naturally-occuring nucleobases, sugars and covalent intersugar (backbone) linkages as well as oligonucleotides having non-naturally-occurring portions which function similarly. Such modified or substituted oligonucleotides are often preferred over native forms because of desirable properties such as, for example, enhanced cellular uptake, enhanced affinity for nucleic acid target and increased stability in the presence of nucleases. A discussion of antisense oligonucleotides and some desirable modifications can be found in De Mesmaeker et al., Acc. Chem. Res., 1995, 28, 366.

The oligonucleotides in accordance with this invention preferably comprise from about 8 to about 30 nucleotides. It is more preferred that such oligonucleotides comprise from about 12 to 25 nucleotides. As it is known in the art, a nucleotide is a base-sugar combination suitably bound to an adjacent nucleotide through a phosphodiester, phosphorothioate or other covalent linkage.

The oligonucleotides used in accordance with this invention may be conveniently and routinely made through the well-known technique of solid phase synthesis. Equipment for such synthesis is sold by several vendors including, for example, Applied Biosystems (Foster City, Calif.). Any other means for such synthesis known in the art may additionally or alternatively be employed. It is also known to use similar techniques to prepare other oligonucleotides such as the phosphorothioates and alkylated derivatives.

In the context of this invention, "hybridization" means hydrogen bonding, which may be Watson-Crick, Hoogsteen or reversed Hoogsteen hydrogen bonding, between complementary nucleotides. For example, adenine and thymine are complementary nucleobases which pair through the formation of hydrogen bonds. "Complementary" as used herein, refers to the capacity for precise pairing between two nucleotides. For example, if a nucleotide at a certain position of an oligonucleotide is capable of hydrogen bonding with a nucleotide at the same position of a DNA or RNA molecule, then the oligonucleotide and the DNA or RNA are considered to be complementary to each other at that position. The oligonucleotide and the DNA or RNA are complementary to each other when a sufficient number of corresponding positions in each molecule are occupied by nucleotides which can hydrogen bond with each other. Thus, "specifically hybridizable" and "complementary" are terms which are used to indicate a sufficient degree of complementary or precise pairing such that stable and specific binding occurs between the oligonucleotide and the DNA or RNA target. It is understood in the art that an oligonucleotide need not be 100 % complementary to its target DNA sequence to be specifically hybridizable. That is, the complementarity is at least 70%, like 80% or 85%, in particular, 90%, particular preferred 95%, like 98% with the target sequence. An oligonucleotide is specially hybridizable when binding of the oligonucleotide to the target DNA or RNA molecule interferes with the normal function of the target DNA or RNA to cause a loss of utility, and there is a sufficient degree of complementarity to avoid non-specific binding of the oligonucleotides to non-target sequences under conditions in which specific binding is desired, i.e. under physiological conditions in the case of in vivo assays or therapeutics treatment, or in the case of in vitro assays, under conditions in which the assays are performed.

The hybridization preferably takes place under stringent experimental conditions which ensure that the hybridization is sequence specific and not random. Stringent hybridization conditions for nucleic acids are known in the art and are published for example in Molecular Cloning: A. Laboratory Manual, J. Sambrook et al., Cold Spring Harbour, New York, 1998 or in current protocols and Molecular Biology, F.M.A. Asibeö et al., John Whiley and Sons, Inc. New York. Various decrease of stringency of hybridization can be employed. More stringently conditions, the greater the complementarity that is required for duplex formation. Stringency can be controlled by temperature, probe concentration, probe length, ionic strength, time and the like. The skilled person is well aware how to obtain specific primers or probes being complementary to the nucleic acid sequence to hybridize with.

The term "samples" in this text refers to any biological material directly collected from an individual. The biological material can be any whole blood, plasma, serum, lymph, sputum, urine, tissue, in particular, tumor containing tissue and stool.

In a first embodiment, the present invention relates to a method for the detection of EGFRvIII specific nucleic acid molecules in a biological sample comprising the step of
a) providing a biological sample containing nucleic acid molecules;
b) amplification of nucleic acid molecules of said biological sample with oligonucleotide primers specific for the EGFRvIII variant; and
c) detection of the application product,
whereby at least one primer of the forward or reverse primer is an oligonucleotide primer spanning exon 1/exon 8 of the EGFR gene.

As described before, the EGFRvIII variant is characterized in a deletion of exon 2 to exon 7 of the EGFR gene. Hence, an oligonucleotide primer spanning exon 1/exon 8 of the EGFR gene will allow to amplify specifically the EGFRvIII variant. Preferably, the sequence of the oligonucleotide primer comprises the sequence of Seq. ID No. 3 in case the oligonucleotide primer is the forward primer and, in case, the exon spanning oligonucleotide primer is the reverse primer, said primer comprises at least the nucleotide sequence of Seq. ID No. 4.

In this connection, it is clear that a mutation, insertion or deletion may be present in the above mentioned primer as long as specific hybridization or annealing is achieved.

In case the exon spanning primer is the forward primer, the reverse primer is located in exon 8 or downstream thereof. In case, the exon spanning primer is the reverse primer, the forward primer is located in exon 1 or upstream thereof.

By using the exon spanning primer, it is possible to amplify EGFRvIII specifically while wild type EGFR is not amplified. This allows determining EGFRvIII nucleic acid molecules in small amounts even when a multiple of wild type EGFR is present.

A second embodiment of the present invention relates to a method for the detection of EGFRvIII specific nucleotide acid molecules in a biological sample comprising the steps of
a) providing a biological sample containing nucleic acid molecules,
b) a first amplification of nucleic acid molecules of said biological sample with oliognucleotide primers specific for the EGFR gene designed to allow amplification of both wild type EGFR and EGFRvIII molecules;
c) a second amplification of nucleic acid molecules with oligonucleotide primers specific for EGFR for amplification of EGFR nucleic acid molecules obtained in step a);
d) detection of the amplification product obtained in step c) whereby the extension time in the first amplification step is of from 3 to 20 seconds.

While shortening the first amplification step to a time range of from 3 to 20 seconds, preferably 5 to 10 seconds, in particular, preferably to 6 to 7 seconds, it is possible to promote the amplification of short nucleic acid molecules, e.g. it is possible to foster the amplification of the EGFRvIII nucleic acid molecules in comparison to the wild type EGFR nucleic acid molecules, since the EGFRvIII molecules lacks nucleic acids of exon 2 to exon 7. For example, in case the primer set is located in exon 1 and exon 8, respectively, the amplification product of the wild type form is about 1000 base pairs (bp's) long while the amplification product of the EGFRvIII is about 160 bp in size. Thus, shortening the extension time in the first amplification step in a nested PCR to only a few seconds allows the polymerase to process short nucleic acid fragments of about 200 base pair (bp) length and only the truncated variant gets enriched.

In the second amplification step of the nested PCR, the oligonucleotide primers are selected to amplify specifically the EGFR nucleic acid molecules obtained in the first amplification step further. In a preferred embodiment, the primer pair of the second amplification step comprises one primer spanning exon 1/exon 8 of the EGFR gene, thus, enabling specific amplification of the EGFRvIII variant only. In addition, preferably the extension time in the second step is also shortened to 3 to 20 seconds, like 5 to 10 seconds, preferably 6 or 7 seconds.

As discussed above, these oligonucleotide primers spanning the junction of exon 1 and exon 8 enables to amplify specifically the variant form.

Another embodiment of the present invention provides a method for the detection of EGFRvIII specific nucleic acid molecules in a biological sample comprising the steps of
a) providing a biological sample containing ribonucleic acid molecules;
b) incubating said biological sample of a) with a deoxynucleic acid probe specifically hybridizing with a region of exon 2 to exon 7 of the EGFR ribonucleic acid molecule, preferably, under stringing conditions and RNAse H;
c) amplification of nucleic acid molecules with oligonucleotide primers specific for EGFR while the forward oligonucleotide primer is specific for a region of the EGFR nucleic acid sequence located upstream from the position where the probe of step b) hybridized to the EGFR nucleic acid molecule and the reverse oligonucleotide primer is complementary to a region located downstream of the hybridization site of said probe or vice versa;
d) detection of the amplification product.

RNase H is an enzyme that cleaves the RNA in a DNA/RNA duplex producing single stranded DNA. Thus, when incubating the biological sample containing ribonucleic acid molecules with the specific deoxynucleic acid probe hybridizing with a region in exon 2 to exon 7 of the EGFR ribonucleic acid molecule, only RNA of the wild type EGFR will be cleaved while the ribonucleic acid molecules encoding the variant form not containing exon 2 to exon 7 remain uncleaved. Thus, in the amplification step following incubation with RNase H and the deoxynucleic acid probe, only EGFRvIII nucleic acid will be amplified.

Preferably, the incubation of the biological probe with RNase H and a probe specifically hybridizing with a region of exon 2 to exon 7 of the EGFRvIII ribonucleic acid molecule is conducted in the above mentioned methods before performing the amplification step with one primer being an oligonucleotide primer spanning exon 1 and exon 8 of the EGFR gene or performing the nested PCR described above.

If necessary, reverse transcription (RT) is performed before amplification of the EGFR nucleotide acid molecules. In another embodiment reverse transcription and amplification may be conducted in a single step as known in the art. The skilled person is aware of suitable methods for reverse transcription.

Typically, amplification is conducted by polymerase chain reaction technique as described above. Preferably, the PCR is a real time PCR technique allowing simultaneously detection of the amplified product. PCR and real time PCR techniques are well known in the art. The skilled person is well aware of the necessary equipment and labelling of the primer molecules to allow simultaneous detection when conducting real time PCR.

The biological sample containing nucleic acid molecules, like deoxynucleic acid molecules and/or ribonucleic acid molecules, may be any body fluid or tissue. Preferably, the biological sample is selected from the group consisting of whole blood, plasma, serum, lymph, sputum, urine, tissue, in particular, tumor containing tissue and stool.

Particularly preferred the biological sample is whole blood to detect EGFRvIII nucleic acid molecules derived from exfoliated precancerous or cancerous lesions. A detection method or molecular screening test based on a blood sample from a patient would abrogate the need for invasive and expensive screening test used today, e.g. the use of antibodies specifically recognizing the EGFRvIII variant. Moreover, the use of the methods according to the present invention allows detecting specifically the EGFRvIII variant more accurate. Even one cell out of 1 million cells may be detected.

Further, the detection of the presence of EGFRvIII allows predicting the responsiveness to EGFR-inhibitors.

Thus, in another aspect of the present invention the method allows the diagnosis of cancer or precancerous lesions comprising the detection of EGFRvIII nucleic acid molecules in a biological sample of an individual suspected to have cancer or having cancer or cancerous lesions.

In another aspect of the present invention, a method for the stratification of an individual for the treatment of cancer to determine whether the cancer or precancerous cells of said cancer will be susceptible to an EGFR inhibitor therapy is provided. Said method for the stratification of an individual comprises a) determining the presence, preferably, the amount, of EGFRvIII nucleic acid molecules in a biological sample of an individual; b) comparing the result of a) with the result determine using a negative control sample or with an already known reference value; and c) determining the susceptibility of the cancer cells to an EGFR inhibitor therapy.

In the above mentioned methods the detection of EGFRvIII specific nucleic acid molecules is indicative of the presence of precancerous or cancerous lesions.

That is, detection of EGFRvIII expression in biological samples is indicative for the presence of precancerous or cancerous cells since EGFRvIII expression is described in precancerous and cancerous cells only. In particular, the method of the present invention allows detecting and, eventually, diagnosing exfoliated cells or metastasizing cells, i.e. few abnormal cells, among millions of normal cells in a sample of an individual, like whole blood.

The cancer may be any one of colon, lung, prostate, oral cavity, oesophagus, stomach, uterus cervix, bladder, breast or glioblastoma. For example, it is known that more than 70 % of breast and ovarian cancers express EGFRvIII, 15 % of lung cancers and 100 % of metastatic prostate cancers.

The method of the present invention may not only allow diagnosing cancer or precancerous lesions but also are useful for the stratification of individuals for the treatment of cancer and to make a decision on the therapy regimen. Further, the claimed methods are useful to control the progress of therapy.

The detection of the amplification product, namely, the EGFRvIII nucleic acid molecules may be achieved by techniques known in the art. For example, detection conducted directly during amplification, e.g. with real-time PCR and melt curve analysis using e.g. cyber green as labelling agent.

In this connection, the primer may be labelled with labelling agents known in the art and depending on the detection method. For example, the primer molecules may be labelled with compounds allowing a detectable signal. The term "label", as used herein, refers to any moiety that functions to: (i) provide a detectable signal; (ii) interact with a second label to modify the detectable signal provided by the first or second label, e.g. FRET; (iii) affect mobility, e.g. electrophoretic mobility, by charge, hydrophobicity, shape, or other physical parameters, or (iv) provide a capture moiety, e.g., affinity, antibody/antigen, or ionic complexation, resulting in degenerated nucleic acid sequences.

Suitable are all kinds of labels and functional groups, such as fluorescent molecules, radioactive molecules, luminescent molecules, enzymes, toxins, dyes, metal particles, magnetic particles, polymer particles, biotin or other organic molecules. Examples of fluorescent molecules are fluorophores such as fluorescein, Texas red, rhodamine, BODIPY, cyber-green, etc, fluorescent proteins or fragments or derivatives thereof such as green fluorescent protein, red fluorescent protein, blue fluorescent protein, renilla fluorescent protein, etc. Examples of radioactive isotopes suitable as label or functional group are phosphorous-32 or -33, sulphur-35, iodine-125, tritium, carbon-14, calcium-45, chromium-51 and all other known radioisotopes which can be coupled to nucleic acids. Examples of enzymes are horse reddish-peroxidase, alkaline phosphatase, luciferase, galactosidase, etc. Suitable toxins are all kinds of toxins such as microbial toxins, toxic peptides or toxic small organic molecules, synthetic toxins or toxins used for medical purposes. An example of a suitable dye is Digoxigenin. Examples of particles are of various particle diameters, preferably between 0.1 to 100 µm. Metal particles are particles which can be made from various substances, preferably heavy elements such as gold, silver, platinum or other suitable metals or alloys or mixtures thereof. Examples of magnetic particles are particles preferably made from all kind of magnetic or magnetizable materials including iron, preferably iron oxides such as Fe₃O, Fe₂O₃, Fe₃O₄, etc. Examples of polymer particles are particles made from a polymer such as polyacrylamide, agarose, polypropylene, polystyrene, polytetrafluoroethylene, etc. Various other labels or functional groups, such as biotin, chitin, maltose or glutathione etc. as known in the art, can also be used.

In a particular embodiment of the present invention, the claimed method is part of a multiplex PCR. In particular, the multiplex PCR additionally comprises the detection of other tumor markers, like CK18, CK19, CK20, MN/CA9, PSA, CA125, CEA, hK2, TG, TSHR, hMAM, NSE and Pmel17 Thus, it is possible to obtain a tissue or individual specific profile of the cancerous lesions the individual is afflicted with.

In another aspect of the present invention, detection kits for detecting EGFRvIII specific nucleic acid molecules in a biological sample are provided. These detection kits comprise a pair of primers wherein at least one primer is designed to be an oligonucleotide primer spanning exon 1/exon 8 of the EGFR gene.

In a further embodiment, the detection kit for detecting EGFRvIII specific nucleic acid molecules in a biological sample comprises RNase H, a probe specifically hybridizing to ribonucleic acid residues present in the region of exon 2 to exon 7 of the EGFR ribonucleic acid molecule.

Preferably, the detection kit further contains a nucleic acid polymerase, dNTP, buffer etc. to perform PCR. In case RT-PCR should be conducted, the kit contains additionally a reversed transcriptase and suitable primers to allow reverse transcription of the ribonucleic acid molecules.

If required, the kit further contains a sample containing EGFRvIII nucleic acid molecules as a positive control and/or instructions to use the kit.

The test kits according to the present invention, allow to determine the absolute concentration or the relative concentration or the presence or absence of a nucleic acid encoding EGFRvIII. The test kit can be used to diagnose the absence or presence of cells expressing EGFRvIII. Further, the test kit can be used to predict the occurrence or to predict the grade or stage of the cancer and/or to predict and/or monitor the success of a therapy for said cancer and/or predict and/or monitor a relapse of said cancer.

Preferably, the test kit can be used to early diagnose and/or predict said cancer, preferably before the diagnosis is possible by other diagnostic tests which are known in the art.

Furthermore, as indicated above, the test kit of the invention can comprise instructions how to use the test kit, how to prepare the samples, what kind of samples to use, how to analyze and interpret the results, etc. especially, the test kit can comprise instructions, how to use the test kit for detection of the presence or absence of cancer cells expressing EGFRvIII variant. The instruction for the test kit can be in written form, presented as pictures, films, audio, computer-multimedia or any other formats suitable to explain, how to use the kit.

Typically, the method according to the present invention starts with a biological sample directly obtained from the individual for example, the biological sample may be whole blood immediately after collection or tissue, e.g. tissue derived from a biopsy or obtained after surgery.

The sample is treated with a solution inactivating RNases inhibiting RNA degradation. Afterwards, the RNA may be isolated by conventional methods, e.g. phenol-chlorofonn extraction or by column chromatography methods known in the art.

Optionally, RNAse H treatment may be conducted. Subsequently, RT-PCR as a one step reaction or two step reaction may be performed according to known protocols and methods. Preferably, the PCR is a real time PCR allowing simultaneous detection of the amount of EGFRvIII nucleic acid molecules.

To conclude, the present invention provides new mutants and kits allowing the detection of EGFRvIII variant in biological sample, like whole blood. The increased sensitivity and specificity of the methods according to the present invention enables to provide reliable molecular diagnostics tests useful in diagnosis and stratification of cancer, including individuals recovery or potential to develop metastatic diseases.

While the present invention has been described above in terms of specific embodiments, it is to be understood that the invention is not limited to these disclosed embodiments. Many modifications and other embodiments of the invention will come to mind of those skilled in the art to which this invention pertains, they are intended to be and are covered by both this disclosure and the appended claims. It is intended that the scope of the invention be determined by proper interpretation and construction of the appended claims and their legal equivalents, as understood by those skilled in the art relying upon the disclosure in this specification and the attached drawings.

### Examples

### Nested PCR comprising two amplification steps

To identify EGFRvIII in biological samples, cDNA was obtained from bloodsamples from 7 healthy donors and patients suffering from ovarian cancer. cDNA was obtained by methods known in the art including reverse transcription of mRNA.

In the first step of the PCR amplification on a BioRad iCycler, the following primer pair was used:

| | |
|---|---|
| Forward primer: | GGGCTCTGGAGGAAAAGAAA (Seq-ID. No. 9) |
| Reverse primer: | GCCCTTCGCACTTCTTACAC (Seq-ID. No. 6) |

To avoid amplification of wtEGFR, the extension time was reduced to a minimum. The polymerase only processes about 200 bp when applying an extension time of about 7 seconds which is nearly the length of the truncated product. The following cycling conditions are used:

| | |
|---|---|
| Cycles: | 30 rounds |
| Denaturation: | 95°C, 20 sec, |
| Annealing: | 57°C, 20 sec, |
| Extension: | 72°C, 7 sec. |

In the second amplification step, 1 µl of the sample obtained in the first step are added to a PCR mixture containing the following primer pair:

| | |
|---|---|
| Forward primer, | GCTCTGGAGGAAAAGAAAGG (Seq-ID. No. 5) |
| Reverse primer: | GCCCTTCGCACTTCTTACAC (Seq-ID. No. 6) |

The cycling conditions are as follows:

| | |
|---|---|
| Cycles: | 45 rounds |
| Denaturation: | 95°C, 20 sec, |
| Annealing: | 57°C, 20 sec, |
| Extension: | 72°C, 7 sec. |

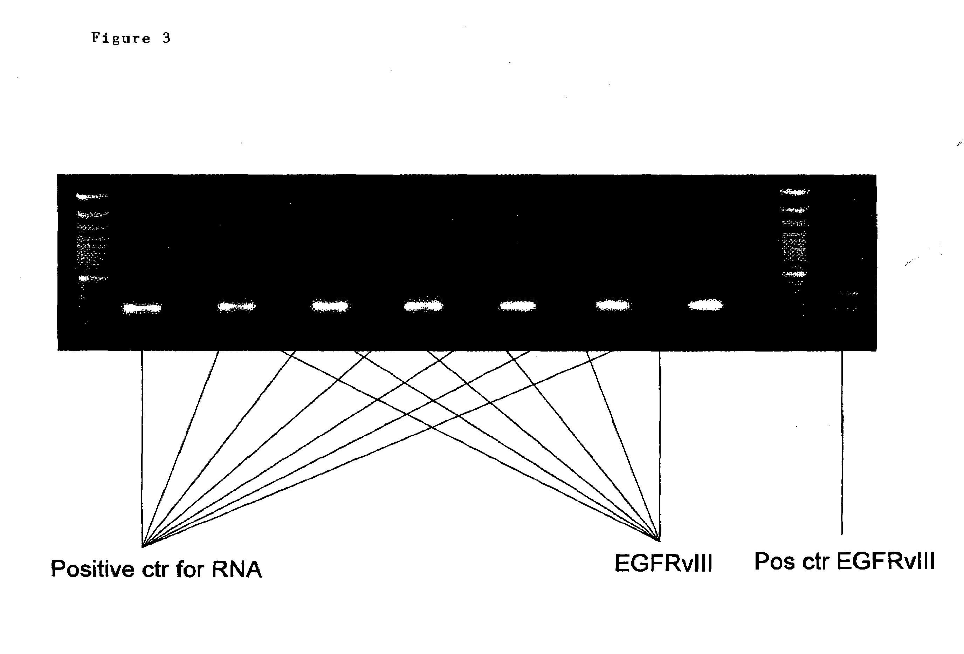

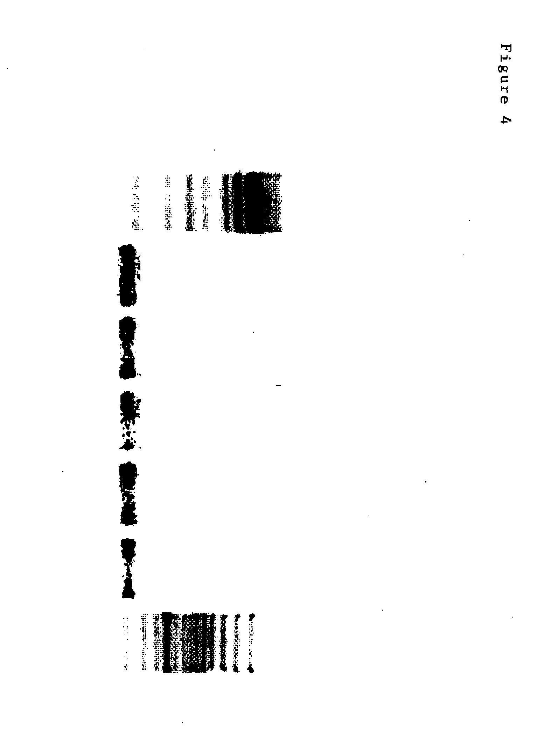

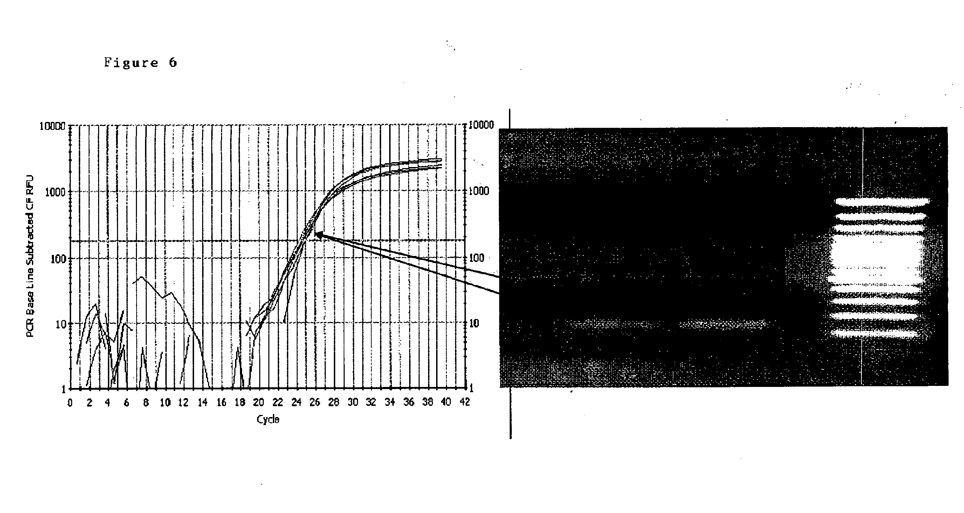

The samples obtained were analysed either directly based on melt curve analysis or on an agarose gel as known in the art. Results are shown in figure 3 for patients suffering from ovarian cancer and figure 4 showing the results in healthy persons. As a positive control cDNA from a brain tumor patient with known EGFRvIII expression was used in figure 4. Figure 6 is from an EGFRvIII positive ovarian cancer patient showing that specific detection of the EGFRvIII variant with real time PCR is possible.

### Detection of EGFRvIII comprising RNaseH treatment.

RNA was isolated by known methods using the RNeasy kit (qiagen, Germany) following manufacturer's instructions. 3 µg RNA was mixed with RNAse H buffer (Invitrogen, USA) and 1 pmol of the following probes were added:
AGCTCCTTCAGTCCGGTTTT (Seq- ID. No. 7) and
GTGGTTCTGGAAGTCCATCG (Seq-ID. No. 8).

These probes hybridize to, exon 3 and 4 of the wild type EGFR, respectively.

Next, the mixture was heated to 80°C for 20 sec and put on ice. 5 Units of RNaseH (Invitrogen, USA) was added and incubated for 10 minutes at room temperature. After incubation, the RNA was cleaned by RNeasy kit. The RNA obtained was reverse transcribed and analysed by the PCR method described above.

## Claims

1. Method for the detection of EGFRvIII specific nucleic acid molecules in a biological sample comprising the step of
a) providing a biological sample containing nucleic acid molecules;
b) amplification of nucleic acid molecules of said biological sample with oligonucleotide primers specific for the EGFRvIII variant; and
c) detection of the amplification product,
whereby any one of the forward or reverse primer is an oligonucleotide primer spanning exon 1/exon 8 of the EGFR gene.

2. Method for the detection of EGFRvIII specific nucleic acid molecules in a biological sample, comprising the steps of
a) providing a biological sample containing nucleic acid molecules,
b) a first amplification of nucleic acid molecules of said biological sample with oligonucleotide primers specific for the EGFR gene designed to allow amplification of both wild-type EGFR and EGFRvIII molecules;
c) a second amplification of nucleic acid molecules with oligonucleotide primers specific for EGFR for amplification of EGFR nucleic acid molecules obtained in step a);
d) detection of the amplification product obtained in step c) whereby the extension time in the first amplification step and, optionally, in the second amplification step, is of from 3 to 20 seconds.

3. Method for the detection of EGFRvIII specific nucleic acid molecules in a biological sample comprising the steps
a) providing a biological sample containing ribonucleic acid molecules;
b) incubating said biological sample of a) with a deoxynucleic acid probe hybridizing with a region of exon 2 to exon 7 of the EGFR ribonucleic acid molecule, in particular under stringent conditions, and RNase H;
c) amplification of nucleic acid molecules with oligonucleotide primers specific for EGFR whereby the forward oligonucleotide primer is specific for a region of the EGFR nucleic acid sequence located upstream from the position where the probe of step b) hybridized to the EGFR nucleic acid molecule and the reverse oligonucleotide primer is complementary to a region located downstream of the hybridization site of said probe or vice versa;
d) detection of the amplification product.

4. The method according to any one of claims 1 to 3 wherein the nucleic acid molecule is a DNA molecule.

5. The method according to any one of the preceding claims wherein the method further comprises the step of reverse transcription of RNA present in the biological sample.

6. The method according to any one of claims 1 to 5, wherein the biological sample is selected from the group consisting of whole blood, plasma, serum, lymph, sputum, urine, tissue, in particular, tumor containing tissue and stool.

7. The method according to any one of the preceding claims, wherein the amplification is conducted by polymerase chain reaction technique.

8. The method according to any one of the preceding claims wherein amplification and detection is conducted by real time PCR technique.

9. The method according to any one of claims 2 to 8, wherein the forward nucleotide primer used in the first amplification step is an oligonucleotide primer comprising a nucleic acid sequence of at least 8 contiguous nucleic acid of exon 1 of the EGFR gene and the reverse primer is an oligonucleotide primer comprising a nucleic acid sequence of at least 8 contiguous nucleic acid complementary to exon 8 of the EGFR gene.

10. The method according to any one of claims 1 to 9, whereby the forward oligo nucleotide primer is an oligonucleotide primer spanning exon 1/exon 8 of the EGFR gene.

11. The method for the detection of EGFRvIII specific nucleic acid molecules in a biological sample according to any one of claims 2 and 4 to 10 wherein in the second amplification step the EGFR specific oligonucleotide primers comprises a forward or reverse primer which is an oligonucleotide primer spanner exon 1/exon 8 of the EGFR gene.

12. The method according to any one of claims 1 or 2 wherein the biological sample containing ribonucleic acid molecules is incubated with a deoxynucleic acid probe hybridizing with a region of exon 2 to exon 7 of the EGFR ribonucleic acid molecule under, in particular stringent conditions, and RNase H before performing the amplification step.

13. The method according to any one of the preceding claims wherein the forward primer is any one of Seq. ID No. 3 or 5 or the reverse primer is any one of Seq.ID No. 4 Or 6.

14. A method for the diagnosis of cancer comprising the step of detecting of EGFRvIII nucleic acid expression according to any one of claims 1 to 13.

15. The method according to claim 14, wherein the cancer is selected from the group consisting of colon, lung, breast, prostate, oral cavity, esophagus, stomach, uterine cervix, breast, glioblastoma or bladder .

16. The method according to any one of claims 14 or 15 for the detection or identification of precancerous lesions or very earlier stage cancer.

17. The method according to any one of claims 14 to 16 for the detection of exfoliated tumor or precancerous cells.

18. A detection kit for detecting EGFRvIII specific nucleic acid molecules in a biological sample, comprising a pair of primers wherein at least one primer is designed to be an oligonucleotide primer spanning exon 1/exon 8 of the EGFR gene.

19. A detection kit for detecting EGFRvIII specific nucleic acid molecules in a biological sample comprising RNase H, and a probe specifically hybridizing to nucleic acid residues present in the region of exon 2 to exon 7 of the EGFR nucleic acid molecule.

20. The detection kit according to any one of claims 18 or 19, further comprising a nucleic acid polymerase, dNTP, and buffer to perform PCR

21. The detection kit according to claim 20 further comprising a reverse transcriptase and suitable primer.

22. The detection kit according to any one of claims 18 to 21 further comprising a sample containing EGFRvIII nucleic acid molecules as a positive control and instructions to use the kit.
